# EUROPEAN PATENT APPLICATION

(11) **EP 4 427 659 A1**
(43) Date of publication of application: **11.09.2024**
(21) Application number: 23160518.9
(22) Date of filing: 07.03.2023
(51) Int. Cl.: A61B 1/00, A61B 1/04, A61B 1/045, A61B 1/06

(54) **DEVICE AND METHOD FOR MEDICAL IMAGING**

(71) Applicant: Erbe Vision GmbH, 78573 Wurmlingen (DE)
(72) Inventor: Shirish, Joshi, 78573 Wurmlingen (DE); Faisal, Kalim, 72764 Reutlingen (DE)
(74) Representative: Rüger Abel Patentanwälte PartGmbB

(57) **Abstract**

The invention relates to a method and a device (1) for medical imaging, in particular intra-operative imaging of an area to be observed of a patient's body. In the invention, fluorescence and live images of the area to be observed are acquired by an imaging unit (3). A processing unit (4) is configured to adapt one or more image capturing or image processing parameters if the images are acquired in a static motion state. This reduces the image noise in the fluorescence images such that the processing unit is able to detect a region of interest in the fluorescence images. The fluorescence images and the corresponding live image depict the same sceneries. The processing unit maps the region of interest detected in the fluorescence images into the live images. If the images are acquired in a moving motion state, the region of interest may be tracked by the processing unit in the live images and mapped back into the fluorescence images. In the moving state, the processing unit is configured to apply a spatially selective noise filter which distinguishes between the region of interest and the remaining region enhancing the quality of the fluorescence images without erasing details with noise filtering in the fluorescence images.

## Description

The invention generally relates to methods, devices, systems and apparatuses for medical imaging, in particular intra-operative imaging of an area to be observed of a patient's body.

Intra-operative medical imaging systems such as endoscopic or laparoscopic imaging systems typically comprise camera systems adapted to be placed close to or within a patient's body for capturing live images during the medical intervention or the surgery. Usually the live images are captured in the visible light range such as having wavelengths in the range of 400 to 780 nm. In the tissue of the patient's body, there may be fluorescent features present that emit fluorescent light if excited by excitation light. The fluorescent light emitted by the fluorescent features may be captured in the live images, however, the fluorescent light is usually weak in the live images. Accordingly, intra-operative medical imaging systems typically comprise fluorescence camera sensors for capturing fluorescence images in the wavelength range at least a major part of the light emitted by the fluorescent features is expected to be, such that the fluorescent features are more clearly visible. The wavelength of the fluorescent light may be situated at least partly in the visible range and at least partly in the infrared light range such as in a wavelength range of 780 nm to 1000 nm.

US patent US 10 694 152 B2 describes an endoscopic video system with a camera comprising a single color image sensor for fluorescence and color imaging, wherein the fluorescence and color images are displayed simultaneously. The tissue under examination is illuminated continuously with fluorescence excitation light and is further illuminated periodically using visible light outside of the fluorescence excitation wavelength range.

In US patent application US 2019/167083 A1, an endoscopic system is described which includes a light source apparatus configured to emit excitation light for exciting a fluorescence medical agent and illuminating light. The endoscopic system further includes an image sensor configured to acquire an image of fluorescence emitted in response to the excitation light from the fluorescent medical agent and reflected light emitted in response to application of the illuminating light. A combined image is generated by combining a first image of the reflected light with a second image of the fluorescence light in a set of pixel arrays that do not overlap each other and each have periodicity.

In US 5 667 474 A, a field sequential image pickup apparatus which inter alia improves the signal-to-noise ratio of an infrared image with respect to movements is described. A movement detection circuit detects a movement in the picture signals. If no movement is detected in the image, a noise reduction operation is executed. On the other hand, if a movement is detected, the noise reduction operation is suppressed.

In JP 2012-085917 A, a method for enhancing the image quality of a fluorescence image by frame addition of a plurality of fluorescence image frames is described. The number of frames added for forming the resulting fluorescence image depends on the detected motion.

Moreover, JP 2002-336187 A, JP H-07250804 A and JP 2012-085916 A describe further methods for enhancing the quality of fluorescence images and for noise reduction.

Still, fluorescence signals in intra-operative live images are usually weaker than the visible light signal. Accordingly, a relatively high amplification of the fluorescence signal is required when visualizing fluorescence light images and visible light images in one overlaid image. However, the relatively high amplification of the fluorescence signal results in an amplification of the noise contained in the fluorescence signal as well.

Therefore, it is an objective of the present invention to provide an improved device and method for medical imaging with an enhanced representation of florescent features.

The objective is solved by the device for medical imaging according to claim 1 and the method for medical imaging according to claim 15:

The inventive device for medical imaging, in particular intra-operative imaging, of an area to be observed of a patient's body comprises at least one illumination source for illuminating the area to be observed, an imaging unit configured to capture live images and fluorescence images of the illuminated area, a processing unit communicatively connected to the imaging unit and a display unit. The at least one illumination source is configured to illuminate the area to be observed with illumination light and/or excitation light.

The illumination light may be in the visible range of light, whereas the excitation light may be in the range of visible light or even in the range of invisible light such as ultraviolet or infrared light. The excitation light may for instance comprise wavelengths that are larger than 500 nanometers or larger than 600 nanometers, an example ranging from 600 to 800 nanometers, 600 to 900 nanometers or 650 to 1350 nanometers (which means in the near fluorescence range). The excitation light may for example also comprise wavelengths that are at least 1400 nanometers, at least 3000 nanometers, at least 8000 nanometers, or at least 15000 nanometers.

The imaging unit may have a visible light image sensor which is configured to capture live images, in particular in the entire visible light range, and a fluorescence image sensor which is configured to capture fluorescence images of the illuminated area, in particular in a specific light range. The imaging unit may for example comprise a dichroic prism that splits the light which enters the imaging unit into a visible light image which is captured by the visible light image sensor and a fluorescence image which is captured by the fluorescence image sensor.

The processing unit is configured to determine whether the live images and/or fluorescence images are in a static motion state. The processing unit may comprise a motion state module configured to determine the motion state of live and/or fluorescence images. For determining the motion state, the processing unit may evaluate changes in the image data of the imaging unit for instance by applying and evaluating the optical flow or comparing particularly significant image features in-between frames. Additionally or alternatively, the processing unit may receive and evaluate motion data from an inertial measurement unit (IMU) which can be located in the camera head. The processing unit is further configured to adapt one or more image capturing or image processing parameters for one or more fluorescence images captured in a static motion state. For example, the processing unit may include a parameter adaptation module for adapting one or more image capturing or image processing parameters for one or more fluorescence images.

Motion in the live and/or fluorescence images can occur due to movement of the imaging unit relative to the tissue, movement of the tissue relative to the imaging unit, or both combined. A static motion state can be defined by an absolute value of the motion, which can be computed in the evaluation of the image data received from the imaging unit and/or the motion data received from the IMU. If the absolute value of the motion is below at least one predetermined threshold, the processing unit may determine that the motion state of the imaging unit is static. If the absolute value of the motion is above the predetermined threshold, the processing unit may determine that the imaging unit is in a moving motion state. The image capturing parameters are for instance the shutter speed of the image sensors, the amplification factor of the imaging sensors and/or the exposure time of the imaging sensors. An image processing parameter is for instance the number of frames that is used for averaging previous frames in order to reduce image noise. The processing unit is configured to detect a region of interest in the fluorescence images captured in a static motion state and map the region of interest into the corresponding live image. The live image or a copy thereof in which the region of interest is mapped is displayed by the display unit.

An aspect of the present invention is based on the idea that the processing unit is able to temporarily adapt one or more image capturing or image processing parameters in case the live images or the live images and the fluorescence images are in a static motion state such that the image noise can be reduced. For instance, the processing unit can decrease the shutter speed and/or the amplification factor such that fluorescence images with a long exposure time, low noise and a high signal-to-noise ratio can be obtained. In this situation, it is crucial that the imaging unit stays in a static motion state, otherwise the fluorescence images will be blurred. In these low noise or high signal-to-noise ratio respectively images, the processing unit can detect a region of interest in the fluorescence images since the image noise is greatly reduced and significant features in the fluorescence images are significantly less blurred. The fluorescence images and the corresponding live image depict the same sceneries which results in a relatively simple mapping of the region of interest and to the corresponding live image. In the fluorescence images fluorescent features of the patient's body in the area to be observed that emit fluorescence light after illuminating the area to be observed with illumination and/or excitation light can be clearly seen, even if hidden behind other layers of biological tissue or obscured by blood or other body fluids.

Fluorescence images can be images that collect only light in a predetermined, preferably narrow-band, wavelength range. The wavelength collected in the fluorescence images by be in the visible light range and/or the invisible light range. If the fluorescence images are in the visible light range, they may for example comprise wavelengths larger than 500 nm. If the fluorescence images are in the infrared light range, they may for example comprise wavelengths that are larger than 600 nm, e.g. ranging from 600 to 800 nm, 600 to 900 nm, or 650 to 1350 nm (i.e. the near-infrared (NIR) range). The infrared light may for example also comprise wavelengths that are at least 1400 nm, at least 3000 nm, at least 8000 or at least 15000 nm.

The processing unit may include a detection module which is configured to detect a region of interest in the fluorescence images, for example by feature detection. The detection module may for instance detect a fluorescing object in the fluorescence images by finding certain intensity values, shapes and the like and defining it as being the region of interest. The region of interest can be for instance described as a bounding box which tightly fits around the detected object in the fluorescence image.

Moreover, the processing unit can be configured to track a previously detected region of interest and subsequent live images if the imaging unit is in a moving motion state. The processing unit is further configured to map the tracked region of interest back into subsequent fluorescence images. For example, the processing unit can comprise a tracking module which is configured to track the region of interest in the live images. When the imaging unit is in a moving motion state, the processing unit is configured to adapt the one or more image capturing or image processing parameters of the fluorescence images accordingly. For instance, the shutter speed or the amplification factor respectively can be increased. This results in an increase of noise in the fluorescence images. Accordingly, a noise reduction algorithm is necessary to be applied to the fluorescence images. Remapping the tracked region of interest into subsequent fluorescence images increases the noise reduction capabilities of the noise reduction algorithm.

In particular, the processing unit is configured to spatially selective noise filter the fluorescence light image, wherein the region of interest is filtered differently than the remaining regions of the fluorescence light image. This may result in better noise reduction results.

For instance, the processing unit is configured to apply a first noise filter in the remaining regions and a separate second noise filter on the region of interest of the fluorescence image in the spatially selective noise filtering. For example, the first noise filter is a running average filter in which the remaining regions of one or more previous frames of the fluorescence images are included exclusively. This allows for avoiding that the detected region of interest is blurred when averaging several previous frames. For instance, the region of interest can be masked when applying the first noise filter on the remaining regions.

Additionally, the first noise filter can be a medium average filter and/or a weighted running average filter of one or more previous frames of the fluorescence images. Using the detected or tracked region of interest for noise filtering of the fluorescence images helps to avoid losing important details in the fluorescence images during the noise filtering.

The processing unit may preferably be configured to continuously determine the motion state, in particular by evaluating the live images and/or the motion data from the IMU. If a static motion state is detected, the processing unit may adapt the one or more image capturing or image processing parameters for one or more fluorescence images, e.g. by increasing the shutter time, the amplification of the fluorescence images and/or the number of frames of fluorescence images added up for noise reduction, such that the noise in these fluorescence images can be effectively reduced. The region of interested is re-detected in these fluorescence images, once the region of interest is re-detected, it is mapped back into the corresponding live image or a copy thereof as well as tracked in subsequent live images and/or fluorescence images.

Moreover, the processing unit can be configured to determine a saturation level in subsequent live images. If the saturation level exceeds a predetermined threshold, the processing unit can perform the following steps:
Adapt the one or more image capturing or image processing parameters for one or more fluorescence images captured in a static motion state,
Detect the region of interest based on the fluorescence images captured in the static motion state and map the region of interest of the fluorescence images into the corresponding live image. This allows the processing unit to come back into a state in which a fluorescence image of high quality with low noise can be obtained and in which the region of interest can be detected again and mapped into the corresponding live image.

In particular, the processing unit is configured to determine whether the imaging unit is in a static motion state via evaluating the image data received from the imaging unit and/or via evaluating motion data of a motion sensor included in the imaging unit.

In one embodiment, the second noise filter includes data from the current fluorescence image only. This means, the second noise filter does not include any history data of previous fluorescence images so that no trailing effects of the region of interest occur. For example, the first noise filter is a spatial median filter applied to the remaining region of the fluorescence image and/or the second noise filter is a spatial Gaussian filter applied to the region of interest of the fluorescence image. In spatial noise filtering, a window of a predetermined size is sled over the image region on which the noise filter is applied on. The first and the second noise filter both may be Gaussian and median filter, wherein the predetermined size of the windows of the first and the second noise filter deviate from one another. For instance, the window size of the first noise filter may be larger than the window size of the second noise filter.

Alternatively, if the first and the second noise filter include previous frames for averaging, the number of previous frames included in the first noise filter is larger than the number of previous frames included in a second noise filter. This results in a reduced trailing effect around the region of interest since less previous frames are taken into consideration of the second noise filter.

Moreover, the one or more image capturing or image processing parameters include a shutter speed of an fluorescence camera sensor of the imaging unit, an amplification factor for the fluorescence images and/or a number of previous frames used for the noise filtering of the fluorescence image by frame averaging.

Furthermore, the processing unit can be configured to adapt the one or more image capturing or image processing parameters for one or more fluorescence images by:
Decreasing the amplification factor and/or the shutter speed of the image unit if the image unit is in a static motion state and/or
Increasing the amplification factor and/or the shutter speed of the image unit if the imaging unit is in a moving motion state. This allows the image capturing or image processing parameters to be adaptively changed in accordance with the motion state that the imaging unit is in.

In a preferred embodiment, the inventive device further comprises an endoscope or laparoscope with a distal end and a proximal end between which an elongated shaft extends. An inertial measurement unit may be positioned at the distal end of the endoscope or the laparoscope. In the endoscope or the laparoscope, the illumination source can be located at a distal end or the proximal end of the endoscope, wherein the elongated shaft comprises at least one optical channel through which the illumination light and/or excitation light is guided from the light source at the proximal end to the distal end when the light source is located at a proximal end. Moreover, the imaging unit can be located at the distal end or the proximal end of the endoscope, wherein the elongated shaft comprises at least one optical channel through which the light reflected and/or emitted from the area to be observed is guided from the distal end to the proximal end when the imaging unit is located at the proximal end.

The inventive method for medical imaging of an in particular intra-operative imaging of an area to be observed includes illuminating the area to be observed with illumination light and/or excitation light capturing live images and fluorescence light images of the illuminated area, determining whether the imaging unit is in a static motion state adapting one or more image capturing or image processing parameters for one or more fluorescence images captured in a static motion state, detecting a region of interest based on the fluorescence images captured in a static motion state, mapping the region of interest of the fluorescence images into the corresponding live image, and displaying the live image or a copy of the live image in which the region of interest is mapped. The displayed live image can be a modified copy of the original live image, e.g. a processed fluorescence image blended into the live image or a copy of the live image.

All the features and advantages described with respect to the inventive device are equally applicable to the inventive method.

Further details and advantages can be taken from the drawings, the respective description and from the claims as well. Embodiments of the invention are illustrated in the drawings, in which
Figure 1 illustrates a first example for device for medical imaging and an area to be observed,
Figure 2 illustrates another example for the device for medical imaging and the area to be observed,
Figures 3a-3c illustrate examples for fluorescence images, live images and processed live images obtained by the imaging unit and processed by the processing unit,
Figures 4a-4c illustrate examples for fluorescence images, live images and processed live images obtained by the imaging unit and processed by the processing unit,
Figures 5a-5c illustrate examples for fluorescence images, live images and processed live images obtained by the imaging unit and processed by the processing unit, as well as
Figure 7 illustrates the flow diagram of the method for medical imaging.

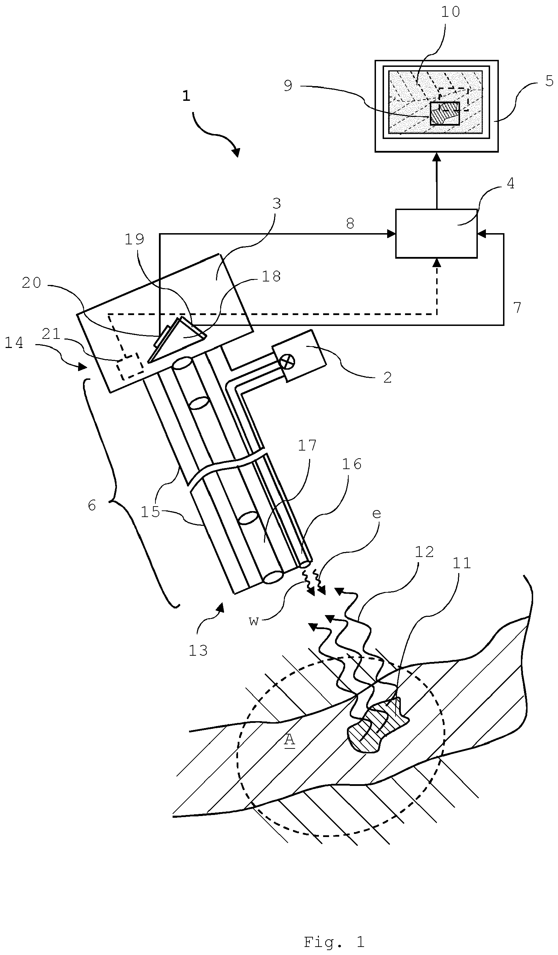

Figure 1 illustrates an example of the device 1 for medical imaging, in particular for intra operative imaging. The device 1 comprises an illumination source 2, an imaging unit 3, a processing unit 4 as well as a display unit 5.

The present example further comprises an endoscope 6 for enabling to capture images inside of a patient's body. Examples with a laparoscope in lieu of the endoscope 6 are also possible.

Figure 1 also shows an area A to be observed of a patient's body, viz. biological tissue. The area A to be observed includes a region 11 that emits fluorescent light when white light w and/or excitation light e shines on this region 11. The endoscope 6 comprises a distal end 13 and a proximal end 14 between which an elongated shaft 15 extends.

The imaging unit 3 is located at the proximal end 14 of the endoscope 6 in figure 1. The illumination source 2 is also located at the proximal end 14 of the endoscope 6. The illumination source 2 is configured to emit white light w and/or excitation light e. As the illumination source 2 located on the proximal end 14 of the endoscope 6, the endoscope 6 provides an optical channel 16 which is configured to guide the white light w and/or the excitation light e from the proximal end 14 to the distal end 13 of the endoscope 6. The white light w and/or the excitation light e is/are emitted from the distal end 13 towards the area A to be observed such that the area A reflects visible light and also fluorescence light 12. The fluorescence light preferable has a wavelength in the range of fluorescence light. Both the visible light and the fluorescence light 12 are captured by an objective 17 at the distal end 13 of the endoscope 6 and guided to the imaging unit 3 which is located at the proximal end 14 of the endoscope 6.

In the imaging unit 3, the reflected visible and fluorescence light 12 is split into a visible light part and a fluorescence light part by a dichroic prism 18. On the dichroic prism 18, a camera sensor 19 for capturing a live image 7 of the visible light part and a fluorescence camera sensor 20 for capturing a fluorescence light image 8 are located on the dichroic prism. Between the camera sensor 19 and the dichroic prism 18 and the fluorescence camera sensor 20 filters may be placed, in order to filter out the parts of the light that should not be captured by each camera sensor 19, 20.

The processing unit 4 is communicatively coupled with the imaging unit 3, such that live images 7 and fluorescence images 8 are transmitted from the imaging unit 3 to the processing unit 4. Moreover, image capturing parameters can be transmitted from the processing unit 4 to the imaging unit 3. For instance, the processing unit 4 can be configured to adapt the image capturing parameters such as the shutter speed, the amplification factor and/or the exposure time. The processing unit 4 is also connected with a display unit 5 which is configured to display a live image 7 and/or a fluorescence image 8 for the operating person.

Furthermore, an inertial measurement unit (IMU) 21 can be located at the proximal end 14of the endoscope 6. The inertial measurement unit 21 can be configured to measure acceleration values and changes in orientation of the inertial measurement unit 21.

The processing unit 4 is configured to determine whether the live images 7 and/or the fluorescence images 8 is/are captured in a static motion state. For determining the motion state in which the live images and/or the fluorescence images are captured, the processing unit 4 may use the image data transmitted from the imaging unit 3 to the processing unit 4 or the measurement data of the inertial measurement unit 21.

The processing unit 4 is further configured to adapt one or more image capturing or image processing parameters for one or more fluorescence images which are captured in a static motion state. The processing unit 4 is also configured to detect a region of interest 9 based on the fluorescence images 8 captured in a static motion state and map the region of interest 9 of the fluorescence images into the corresponding live image 7.

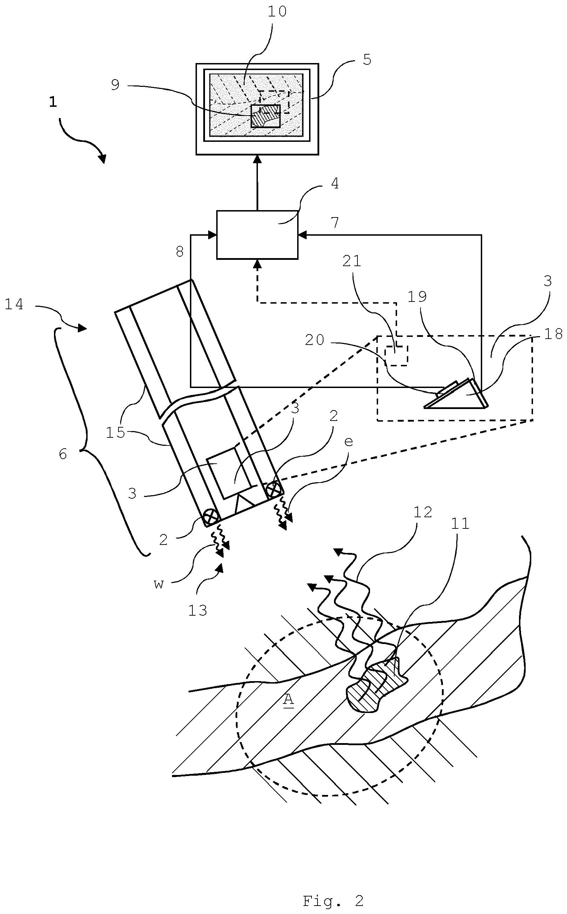

Figure 2 illustrates another example of the device 1 for medical imaging. The example depicted in figure 2 differs from the example depicted in figure 1 in that the illumination source 2 and the imaging unit 3 are both located at the distal end 13 of the endoscope 6. For the example shown in figure 2, the same applies as described with respect to figure 1 based on the reference signs.

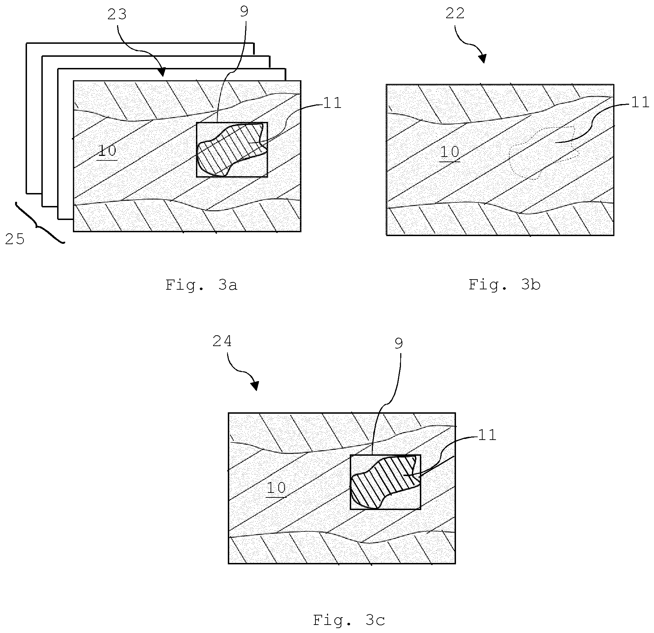

Figures 3a-3c depict a frame 22 at time T of the live images 7, a frame 23 of a plurality of fluorescence images 25 and a resulting frame 24 of the live image 7 in which a region of interest 9 is mapped.

Figure 3a shows frame 22 at time T of the live image 7 gathered from the imaging unit 3. In the frame 22, the region 11 that emits fluorescence light only has a weak signal strength such that the region 11 emitting fluorescence light is barely or not observable in the frame 22. Accordingly, a person operating the device 1 is not able to see the fluorescence region 11 in the live image 7. In this situation, the processing unit 4 is configured to determine whether the live images 7 and/or the fluorescence images 8 are captured in a static motion state. For determining the motion state, the processing unit may use an optical flow method, a block matching method or data from the inertial measurement unit 21 or the like.

If the processing unit 4 determined that the live images 7 and/or fluorescence images 8 are captured in a static motion state, the processing unit 4 adapts one or more image capturing or image processing parameters for one or more fluorescence images 8.

In this example, the processing unit 4 decreases the shutter speed of the fluorescence camera and averages multiple frames 25 of the fluorescence camera sensor 20 which is illustrated in figure 3b. Alternatively, the processing unit 4 may decrease the shutter speed of the fluorescence camera or average multiple frames 25 of the fluorescence camera sensor 20. Regardless of whether the processing unit 4 decreases the shutter speed of the fluorescence camera and/or averages multiple frames 25 of the fluorescence camera sensor 20, this leads to an enhancement of the quality of the resulting fluorescence image 23 such that the region 11 emitting fluorescent light is clearly depicted in the resulting fluorescence image 23. The processing unit 4 is therefore able to detect a region of interest 9 in the resulting fluorescence image 23. The processing unit 4 may for instance use a feature detection algorithm for detecting the region of interest 9. After detecting the region of interest 9, the processing unit 4 maps the region of interest 9 into the corresponding live images 7.

Figure 3c shows a corresponding frame 24 of the live images 7 in which the region of interest 9 is mapped. The display unit 5 is configured to display at least the live images 7 in which the region of interest 9 gathered from the fluorescence images 8 are mapped.

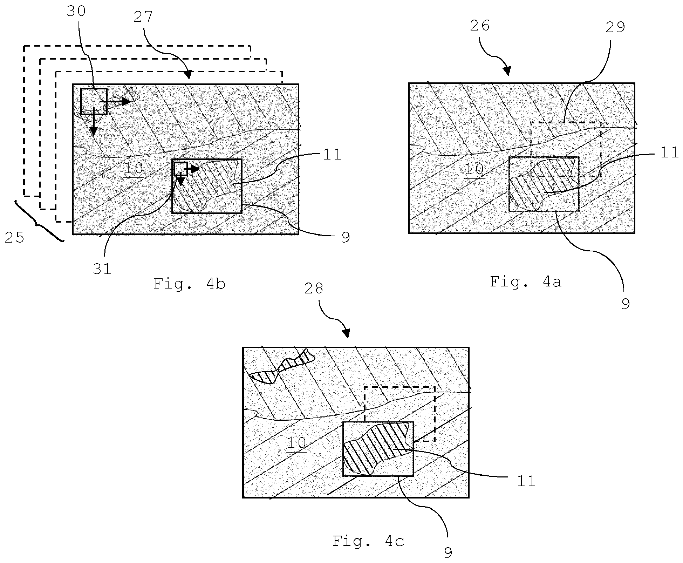

Figures 4a-4c depict a subsequent frame 26 at time T+m of the live images 7, a corresponding frame 27 of the fluorescence images 8 and a frame 28 of the corresponding frame 27 of the fluorescence images 8 after applying the spatially selective noise filter when the live images 7 are in a moving motion state.

In the subsequent frame 26 of the live images 7 in figure 4a, the processing unit 4 tracks the region of interest 9, for example in a region close to previous position 29 of the region of interest 9. After tracking the region of interest 9, the processing unit 4 maps back the region of interest 9 into a corresponding present frame 27 of the fluorescence images 8, which is depicted in figure 4b.

Subsequently, the processing unit 4 is configured to apply a spatially selective noise filter to the fluorescence image 8. In the spatially selective noise filter, the region of interest 9 is filtered differently than the remaining regions 10 of the fluorescence image 8.

In figure 4b, a first noise filter with a filter window 30 of a predetermined window size is sled over the remaining region 10 of the fluorescence light image 8, whereas a second noise filter with a second window 31 of a predetermined window size which is smaller than the window size of the first noise filter is sled over the region of interest 9 only.

Additionally or alternatively, the first noise filter is a frame averaging filter which includes the remaining regions 10 of a plurality of previous frames 25 of the fluorescence images 8.

In figure 4c the resulting frame 28 of the fluorescence images 7 after applying the spatially selective noise filter is illustrated.

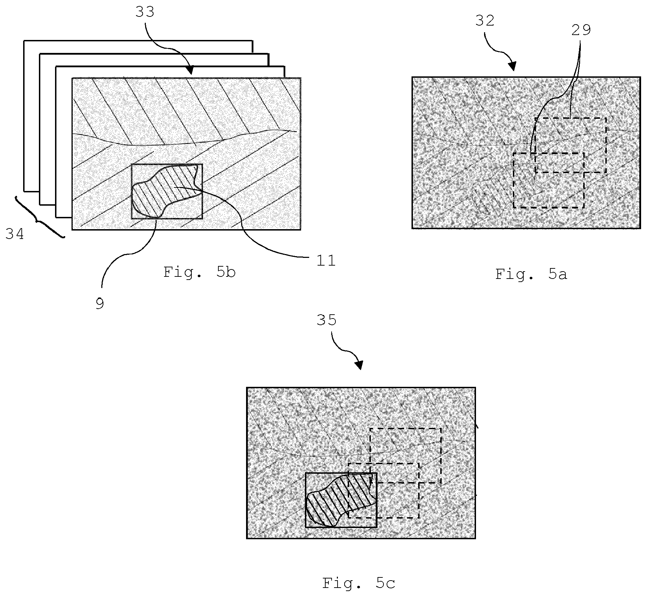

Figures 5a-5c illustrate a further subsequent frame 32 at time T+n of the live images 7 and a frame 33 generated by averaging a plurality of frames 34 of the fluorescence images 8, wherein n is larger than m.

In figure 5a, the saturation level of the current frame 32 of the live images 7 exceeds a certain threshold such that the region of interest 9 cannot be tracked by the processing unit 4 in the current frame of the live images 7.

Accordingly, the processing unit 4 may indicate the operating person that the imaging unit 3 shall now be put into a static motion state. The processing unit 4 may for instance signal the operating person to hold the imaging unit 3 still by showing a sign or a message on the screen of the displaying unit 5. The processing unit 4 determines if the fluorescence images or the live images are captured in a static motion state. If that is the case, the processing unit 4 decreases the shutter speed of the fluorescence camera sensor 20 again, so that the quality of the fluorescence images 8 increases and the region of interest 9 can be detected in a frame 33 generated by averaging the plurality of frames 34 of the fluorescence images 8. After detecting the region of interest 9 in the fluorescence images 8, the processing unit 4 maps the region of interest into the corresponding frame 32 of the live images 7 generating a frame 35 with extra information regarding the fluorescing object, which is illustrated in figure 5c.

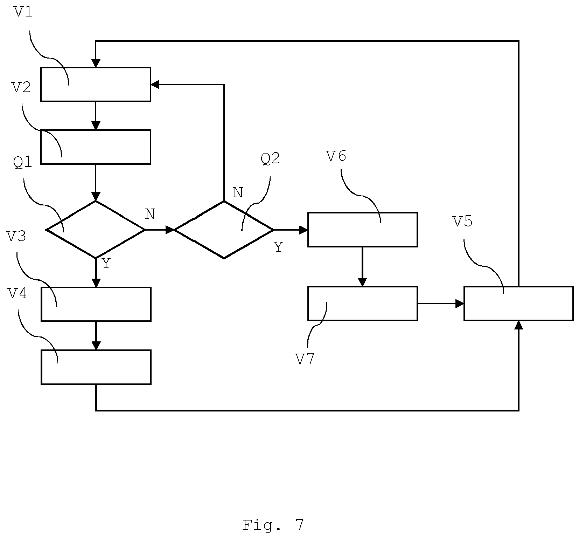

Figure 7 shows a flow diagram of the method for medical imaging, in particular intra-operative imaging.

The method begins in method step V1, in which the area A to be observed is illuminated with illumination light w and/or excitation light e. Then, live images 7 and fluorescence light images 8 of the illuminated area A are captured in method step V2.

After that, it is determined in step Q1 if the live images 7 and/or the fluorescence images 8 are captured in a static motion state. If that is the case, one or more image capturing or image processing parameters are adapted for one or more fluorescence images 8 in method step V3. After adapting the image capturing or image processing parameters, a region of interest 9 is detected in the fluorescence images 8 captured in a static motion state and the detected region of interest 9 is mapped into the corresponding live image 7 in method step V4. The live image 7 in which the region of interest 9 is mapped is displayed for the operating person in method step V5.

If it is determined that the fluorescence 8 or live images 7 are not captured in a static motion state in step Q1, it is determined whether a region of interest 9 was detected in previous frames in step Q2. If that is not the case, the algorithm continues with method step V1. If a region of interest 9 was detected in a previous frame, the algorithm tracks the region of interest 9 in the current frame of the live images and maps the region of interest 9 into the fluorescence image in method step V6. Also, the algorithm applies a spatially selective noise filter to the fluorescence images 8 in method step V7. After that, the algorithm continues with displaying at least the live image 8 in which the region of interest is mapped or tracked in method step V5.

The invention relates to a method and a device for medical imaging, in particular intra-operative imaging of an area to be observed of a patient's body. In the invention, fluorescence and live images of the area to be observed are acquired by an imaging unit. A processing unit is configured to adapt one or more image capturing or image processing parameters if the images are acquired in a static motion state. This reduces the image noise in the fluorescence images such that the processing unit is able to detect a region of interest in the fluorescence images. The fluorescence images and the corresponding live image depict the same sceneries. The processing unit maps the region of interest detected in the fluorescence images into the live images. If the images are acquired in a moving motion state, the region of interest may be tracked by the processing unit in the live images and mapped back into the fluorescence images. In the moving state, the processing unit is configured to apply a spatially selective noise filter which distinguishes between the region of interest and the remaining region enhancing the quality of the fluorescence images without erasing details with noise filtering in the fluorescence images.

### List of Reference Signs:

- 1: Device
- 2: light source
- 3: imaging unit
- 4: processing unit
- 5: display unit
- 6: endoscope
- 7: live images
- 8: fluorescence images
- 9: region of interest (ROI)
- 10: remaining regions
- 11: region emitting fluorescent light
- 12: fluorescence light
- 13: distal end of the endoscope
- 14: proximal end of the endoscope
- 15: elongated shaft
- 16: optical channel
- 17: optical channel (objective)
- 18: dichroic prism
- 19: camera sensor
- 20: fluorescence camera sensor
- 21: inertial measurement unit (IMU)
- 22: frame at time T of the live images
- 23: resulting fluorescence image
- 24: frame of the live images with mapped ROI
- 25: previous frames of the fluorescence images
- 26: subsequent frame of the live images
- 27: corr. pres. frame of the fluorescence images
- 28: resulting frame after spatially selective noise filter
- 29: previous position of the ROI
- 30: filter window of the first noise filter
- 31: filter window of the second noise filter
- 32: frame of the live images
- 33: frame generated by averaging the plurality of frames of the fluorescence images
- 34: plurality of frames of the fluorescence images
- 35: generated frame with extra information
- A: area to be observed
- e: excitation light
- N: no
- Q1: request if live images are in a static motion state
- Q2: request if the ROI has been detected in previous frames
- V1: illuminating the area to be observed
- V2: capturing live and fluorescence images
- V3: adapting the image capture or processing parameters
- V4: detect the ROI and map the ROI into the live image
- V5: display the live image with the ROI mapped therein
- V6: tracking the ROI and mapping the ROI into the fluorescence images
- V7: spatially selective noise filtering
- w: illumination light
- Y: yes

## Claims

1. Device (1) for medical imaging, in particular intra-operative imaging, of an area (A) to be observed comprising:
at least one light source (2) for illuminating the area (A) to be observed with illumination light (w) and/or excitation light (e);
an imaging unit (3) configured to capture live images (7) and fluorescence images (8) of the illuminated area (A);
a processing unit (4) communicatively connected with the imaging unit (3) and configured to:
determine whether the live images (7) and/or the fluorescence images (8) is/are captured in a static motion state,
adapt one or more image capturing or image processing parameters for one or more fluorescence images (8) captured in a static motion state,
detect a region of interest (9) based on the fluorescence images (8) captured in a static motion state, and map the region of interest (9) of the fluorescence images (8) into the corresponding live image (7); and
a display unit (5) configured to display the live images (7) or copies of the live images (7) in which the region of interest (9) is mapped.

2. Device (1) according to claim 1, **characterized in that** the processing unit (4) is configured to track a previously detected region of interest (9) in subsequent live images (7), if the imaging unit (3) is in a moving motion state, and map back the tracked region of interest (9) into subsequent fluorescence images (8).

3. Device (1) according to claim 1 or 2, **characterized in that** the processing unit (4) is configured to spatially selective noise filter the fluorescence light image (8), wherein the region of interest (9) is filtered differently than the remaining regions (10) of the fluorescence light image (8).

4. Device (1) according to claim 3, **characterized in that** the processing unit (4) is configured to apply a first noise filter on the remaining regions (10) and a separate second noise filter on the region of interest (9) of the fluorescence image (8) in the spatially selective noise filtering.

5. Device (1) according to claim 4, **characterized in that** the first noise filter (11) is a running average filter in which the remaining regions (10) of one or more previous frames of the fluorescence images (8) are included exclusively.

6. Device (1) according to claim 4, **characterized in that** the first noise filter (11) is a median average filter and/or a weighted running average filter of one or more previous frames of the fluorescence images (8).

7. Device (1) according to any of the previous claims, **characterized in that** the processing unit (4) is configured to determine a saturation level in subsequent live images (7) and perform the following steps, if the saturation level exceeds a predetermined threshold:
adapt the one or more image capturing or image processing parameters for one or more fluorescence Images (8) captured in a static motion state,
detect the region of interest (9) based on the fluorescence Images (8) captured in a static motion state, and map the region of interest (9) of the fluorescence images (8) into the corresponding live image (7).

8. Device (1) according to any of the previous claims, **characterized in that** the processing unit (4) is configured to determine whether the imaging unit (3) is in a static motion state via evaluating the image data received from the imaging unit (3) and/or via evaluating motion data of a motion sensor (21) included in the imaging unit (3).

9. Device (1) according to any of the previous claims, **characterized in that** the second noise filter includes data from the current fluorescence image (8) only.

10. Device (1) according to any of the previous claims, **characterized in that** the second noise filter (11) is a spatial median based filter applied to the remaining region (10) of the fluorescence image (8) and/or the second noise filter (12) is a spatial Gaussian based filter applied to the region of interest (9) of the fluorescence image (8).

11. Device (1) according to any of claims 1-8, **characterized in that** the number of previous frames included in the first noise filter is larger than the number of previous frames included in the second noise filter.

12. Device (1) according to any of the previous claims, **characterized in that** the one or more image capturing or image processing parameters include a shutter speed of an fluorescence camera sensor of the imaging unit (3), an amplification factor for the fluorescence images (8), and/or a number of previous frames used for noise filtering the fluorescence Image (8) by frame averaging.

13. Device (1) according to claim 12, **characterized in that** the processing unit (4) is configured to adapt the one or more image capturing or image processing parameters (13) for one or more fluorescence Images (8) by:
decreasing the amplification factor and/or the shutter speed of the imaging unit, if the imaging unit (3) is in a static motion state, and/or
increasing the amplification factor and/or the shutter speed of the imaging unit, if the imaging unit (3) is in a moving motion state.

14. Device (1) according to any of the previous claims, further comprising an endoscope (6) with a distal end (13) and proximal end (14) between which an elongated shaft (15) extends,
wherein an inertial measurement unit (21) is positioned at the distal end (13) of the endoscope (6); wherein the light source (2) is located at the distal end (13) or the proximal end (14) of the endoscope (6), wherein the elongated shaft (15) comprises at least one optical channel (16) through which the illumination light (w) and/or excitation light (e) is guided from the light source (2) at the proximal end (14) to the distal end (13) if the light source (2) is located at the proximal end (14);
wherein the imaging unit (3) is located at the distal end (13) or the proximal end (14) of the endoscope (6), wherein the elongated shaft (15) comprises at least one optical channel (17) through which light reflected and/or emitted from the area to be observed (A) is guided from the distal end (13) to the proximal end (14) if the image unit (3) is located at the proximal end (13).

15. Method for medical imaging, in particular intra-operative imaging, of an area to be observed, preferably with a device according to any of the previous claims, including:
illuminating (V1) the area (A) to be observed with illumination light (w) and/or excitation light (e);
capturing (V2) live images (7) and fluorescence light images (8) of the illuminated area (A);
determining (Q1) if the imaging unit (3) is in a static motion state;
adapting (V3) one or more image capturing or image processing parameters for one or more fluorescence Images (8) captured in a static motion state;
detecting (V4) a region of interest (9) based on the fluorescence images (8) captured in a static motion state;
mapping (V6) the region of interest (9) of the fluorescence images (8) into the corresponding live image (7); and
displaying (V7) the live image (4) in which the region of interest (9) is mapped.
